Europäisches Patentamt

European Patent Office

Office européen des brevets

(19)

(11) Publication number: **0 047 906**
**B1**

(12) **EUROPEAN PATENT SPECIFICATION**

(45) Date of publication of patent specification: **13.06.84**

(21) Application number: **81106765.1**

(22) Date of filing: **29.08.81**

(51) Int. Cl.³: **C 07 C 41/06, C 07 C 43/04**

(54) Process for the production of aliphatic ethers.

(30) Priority: **12.09.80 US 186863**

(43) Date of publication of application:
**24.03.82 Bulletin 82/12**

(45) Publication of the grant of the patent:
**13.06.84 Bulletin 84/24**

(84) Designated Contracting States:
**BE DE FR GB IT**

(56) References cited:
**GB - A - 2 049 693**

(73) Proprietor: **PHILLIPS PETROLEUM COMPANY
5th and Keeler
Bartlesville Oklahoma 74004 (US)**

(72) Inventor: **Van Pool, Joe
1418 Prairie Heights Drive
Bartlesville Oklahoma 74003 (US)**

(74) Representative: **Dost, Wolfgang, Dr.rer.nat., Dipl.-
Chem. et al,
Patent- und Rechtsanwälte Bardehle-Pagenberg-
Dost-Altenburg & Partner Postfach 86 06 20
D-8000 München 86 (DE)**

Courier Press, Leamington Spa, England.

## Description

The present invention relates to the production of aliphatic ethers by the catalytic reaction of iso-olefins with alcohols.

It is well known that aliphatic ethers can be formed by reacting an iso-olefin and an alcohol in the presence of a solid acid ion exchange resin. Naturally in a commercial operation it is desirable that the catalyst be maintained effective over long periods of time. This invention is based upon the discovery that many of the iso-olefin containing feedstreams that are used in such processes contain small amounts of basic compounds. Even though the amounts of basic compounds are generally rather low their neutralizing effect upon the etherization catalyst is cumulative.

In accordance with the present invention, the effective life of the acid ion exchange resin is prolonged by reducing the amount of basic material in the iso-olefin feedstream before said iso-olefin feedstream is brought into contact with said acid ion exchange resin.

Accordingly, the invention relates to a process for the production of aliphatic ethers wherein a first feedstream comprising at least one isoolefin having 4 to 16 carbon atoms and a second feedstream comprising at least one alcohol selected from primary and secondary aliphatic alcohols containing 1 to 12 carbon atoms are contacted with an acid ion exchange catalyst which process is characterized by mixing the first feedstream with a diluted aqueous solution of sulfuric acid having a pH of more than 4 and less than 7 at a temperature of 21—49°C before said first feedstream is brought into contact with said acid ion exchange catalyst.

It is known from "Procédés de Pétrochimie", 1971, pages 129—135 to treat $C_4$ isoolefin fractions with concentrated sulfuric acid (50%) at a temperature of about 30°C in order to extract isobutene therefrom. The reference does not deal with the catalytic production of aliphatic ethers by reacting an isoolefin with an alcohol, it is silent on the deterioration of the catalyst by basic compounds contained in the iso-olefin feedstream and it is particularly silent on the removal of the said basic compounds.

Presently, it is preferred that the pretreatment of the isoolefin feedstream be conducted before the alcohol feedstream is combined therewith.

The isoolefins which may be employed in the present invention include any of those having 4 to 16 carbon atoms per molecule which are readily converted to ethers by reaction with primary or secondary alcohols in the presence of the acid ion exchange resin catalyst. Examples of such iso-olefins include isobutylene, isoamylene, isohexylene, isoheptylene, isooctylene, isononylene, isodecylene, isoundecylene, isododecylene, iso-tridecylene, isotetradecylene, isopentadecylene, and isohexadecylene, or mixtures of two or more thereof.

The alcohols which may be utilized include the primary and secondary aliphatic alcohols of from 1 to 12 carbon atoms, such as methanol, ethanol, propanol, isopropanol, the primary and secondary butanols, pentanols, hexanols, ethylene glycol, propylene glycol, butylene glycol, the polyglycols, and glycerol, or mixtures of two or more thereof.

The presently preferred reactants are methanol and isobutylene because they yield methyl tertiary butyl ether (MTBE) which has utility as an octane improver for gasoline. Accordingly it is currently preferred for the iso-olefins to be predominately isobutylene and the alcohols predominately methanol. Even more preferably the iso-olefins consist essentially of isobutylene and the alcohols consist essentially of methanol.

It is generally preferred for the iso-olefin and the alcohol to be passed through the reaction zones in the presence of diluents which do not have an adverse effect upon the etherification reaction. The diluents can be present in either the first stream or the second stream, or both, preferably the diluent is in the iso-olefin stream. Examples of suitable diluents include alkanes and straight chain olefins. The feed to the reactors, excluding alcohol, is generally diluted so as to include from 2 to 80 weight percent iso-olefin, preferably from 10 to 60 weight percent.

Preferably, the isobutylene stream consists essentially of the $C_4$ olefin fraction of a hydrocarbon cracking process, i.e., a fluid catalytic cracking process. Typically, the $C_4$ olefin fraction of such cracking processes will be materials which have been subjected to an aqueous caustic wash to insure substantial removal of sulfur or sulfur compounds. Such compositions, even after a water wash following the caustic wash, often contain basic material in amounts in the range of 0.1 to 20 parts per million by weight. The basic material is generally ammonia resulting from nitrogen compounds in the feed to the catalytic cracker or residual caustic from the caustic wash process.

The isobutylene feedstream can also be provided from other sources such as from a natural gas extraction plant wherein isobutane is dehydrogenated to isobutylene. The isobutylene can be in either the liquid or gaseous state when contacted with the acid. Preferably, the isobutylene is in the liquid phase.

The pretreatment of the iso-olefin feedstream is effected with a dilute aqueous solution of sulfuric acid having a pH of more than 4 and less than 7, most preferably about 5. Although higher concentrations of acid such as 3 weight percent will work, the lower concentrations are preferred since they minimize the buildup of sulfate salts in the treated olefin stream.

The iso-olefin and the acidic aqueous solution can be contacted in any manner which results in a reduction in the basic materials in the iso-olefin. Preferably, the iso-olefin and the acidic solution are so thoroughly mixed that substantially all of the basic material is neutralized. One technique for contacting the iso-olefin and the aqueous acid solution involves the countercurrent contact resulting from passing the iso-olefin through a spray of the acidic solution. Another technique involves combining the iso-olefin and the acidic solution in a mixing eductor, such as an Schutse & Koerting 242 mixing eductor.

It is, of course, preferably that the conditions be selected such that the acidic solution is maintained substantially in the liquid state so that the iso-olefin can be readily separated therefrom. The contacting of the olefin and the acid solution is conducted at temperatures in the range 70 to 120°F (21—49°C), preferably about 95°F (35°C). The iso-olefin is generally introduced to the acidic solution under pressure, for example, typically pressures would be in the range of 30 to 300 psig (2—20.7 bars gauge) preferably about 100 psig (6.9 bars gauge).

The acid ion-exchange catalysts useful in accordance with the present invention are relatively high molecular weight carbonaceous material containing at least one $-SO_3H$ functional group. These catalysts are exemplified by the sulfonated coals ("Zeo-Karb(TM) H", "Nalcite(TM) X" and "Nalcite(TM) AX") produced by the treatment of bituminous coals with sulfuric acid and commercially marketed as zeolitic water softeners or base exchangers. These materials are usually available in a neutralized form and in this case must be activated to the hydrogen form by treatment with a strong mineral acid such as hydrochloric acid and water washed to remove sodium and chloride ions prior to use. The sulfonated resin type catalysts are preferred for use in the present invention. These catalysts include the reaction products of phenolformaldehyde resins with sulfuric acid ("Amberlite(TM) IR-1", "Amberlite(TM) IR-100" and "Nalcite(TM) MX"). Also useful are the sulfonated resinous polymers of coumarone-indene with cyclopentadiene, sulfonated polymers of coumarone-indene with cyclopentadiene and furfural and sulfonated polymers of cyclopentadiene with furfural. The most preferred cationic exchange resins are strongly acidic exchange resins consisting essentially of sulfonated polystyrene resin, for instance, a divinylbenzene crosslinked polystyrene matrix having from 0.5 to 20 percent and preferably from 4 to 16 percent of copolymerized divinylbenzene therein to which are ionizable or functional nuclear sulfonic acid groups. These resins are manufactured and sold commercially under various trade names such as "Dowex(TM) 50", "Nalcite(TM) HCR" and "Amberlyst(TM) 15". As commercially obtained they have solvent contents of about 50 percent and can be used as is or the solvent can be removed first. The resin particle size is not particularly critical and therefore is chosen in accordance with the manipulative advantages associated with any particular size. Generally mesh sizes of 10 to 50 United States Sieve Series (0.3—2 mm) are preferred. The reaction may be carried out in either a stirred slurry reactor or in a fixed bed continuous flow reactor. The catalyst concentration in a stirred slurry reactor should be sufficient to provide the desired catalytic effect. Generally catalyst concentration should be 0.5 to 50 percent (dry basis) by weight of the reactor contents with from 1 to 25 percent being the preferred range.

Acid ion exchange resins, such as Amberlyst(TM) 15, are currently the most preferred catalyst for the etherification.

The temperature for the reaction zones and the space velocity for the feeds to the reactor zones can be selected as desired depending upon the degree of conversion desired and the temperatures at which oligomerization becomes a problem. Generally, the temperature of the reaction zones will be in the range of 30°C to 120°C, preferably 35°C to 80°C. Pressures are generally selected to ensure that the charges and the products remain liquids during the reaction. Typical pressures are in the range of about 30 to about 300 psig (2 to 20.7 bars gauge). Generally, the liquid hourly space velocity (LHSV) of feed in the reactors will be in the range of 5 to 50 $hr^{-1}$, preferably 5 to 20 $hr^{-1}$.

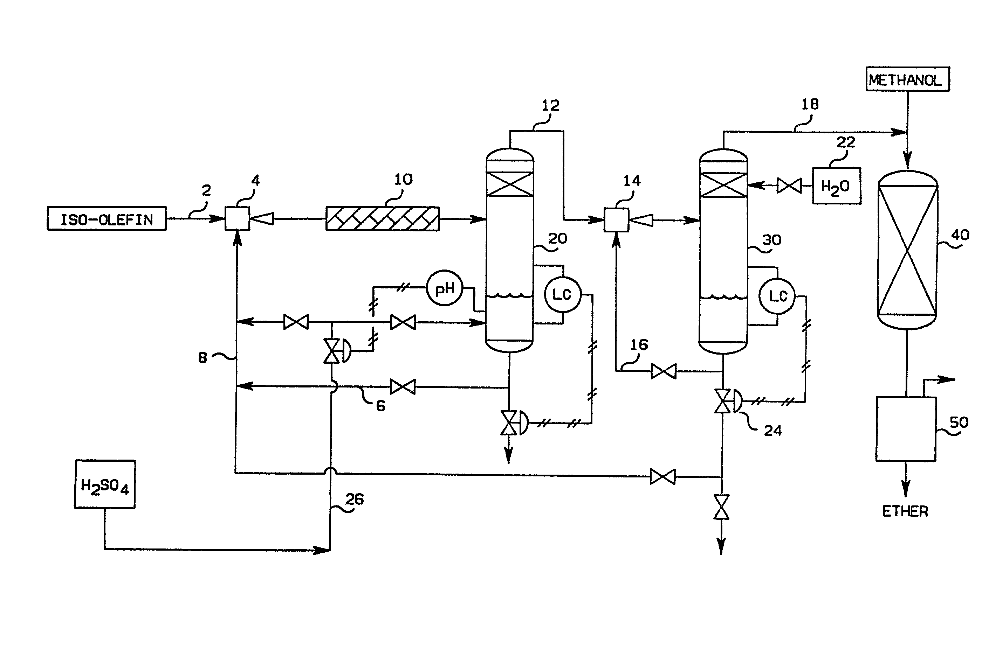

In order to provide a better understanding of the present invention, a preferred embodiment will be described with reference to the accompanying drawing.

The system illustrated in the drawing includes a first separation column 20, a second separation column 30, an etherization reactor 40 filled with a fixed bed of acid ion exchange catalyst, and a separation zone 50. The separation column 20 contains a quantity of dilute aqueous sulfuric acid. The separation column 30 contains a quantity of water.

Iso-olefin from a source of supply, such as the $C_4$ cut of the effluent of a cracking process, is passed via line 2 into a mixing eductor 4. The eductor 4 in turn draws aqueous sulfuric acid from column 20 via lines 6 and 8. The mixture of iso-olefin and acid solution is then passed to a static in-line mixer 10 to effect still further mixing. The mixed stream then passes into column 20 wherein the heavier acid solution separates and the iso-olefin exits overhead through line 12.

The iso-olefin in line 12 in turn is passed through mixing eductor 14. Eductor 14 draws water from column 30 via line 16 for mixing with the iso-olefin. The mixture of isoolefin and water is then passed into column 30 wherein the water separates and the iso-olefin exits overhead through line 18 where it is combined with methanol and fed to the etherification reactor 40. The effluent from the etherification reactor is then passed to a separation zone wherein ether is separated from the other components and recovered.

In operating the pretreatment system, makeup water from a source 22 is continuously or intermittently added to column 30 as necessary to maintain the desired level of water in column 30. A valve 24 operated by a liquid level control in column 30 is included to allow the water of column 30 to be drained off either to column 20 via line 8 or to some other point of disposal.

Column 20 also includes a liquid level control which can be used to control the amount of acid solution that is circulated through eductor 4. Excess acid is released to a point of disposal. Column 20 further includes a pH controller which allows makeup acid to be added via line 26 as necessary to maintain a given pH in column 20. The acid can be added either directly to the column or through eductor 4 as desired.

Although it is not preferred one could dispense with the water wash column 30. Further, one could carry out the present invention with batch rather than continuous treatment of the iso-olefin feed.

## Claims

1. A process for the production of aliphatic ethers wherein a first feedstream comprising at least one isoolefin having 4 to 16 carbon atoms and a second feedstream comprising at least one alcohol selected from primary and secondary aliphatic alcohols containing 1 to 12 carbon atoms are contacted with an acid ion exchange catalyst characterized by mixing the first feedstream with a diluted aqueous solution of sulfuric acid having a pH of more than 4 and less than 7 at a temperature of 21—49°C before said first feedstream is brought into contact with said acid ion exchange catalyst.

2. The process of claim 1 characterized in that said first feedstream consists essentially of the $C_4$ olefin fraction of a hydrocarbon cracking process.

3. The process of claim 2 characterized in that the iso-olefin in said first feedstream consists essentially of isobutylene and said alcohol in said second feedstream consists essentially of methanol.

4. The process of any of claims 1 to 3 characterized in that said acid ion exchange catalyst is an acid ion exchange resin.

5. The process of any of claims 1 to 4 characterized in that said first feedstream contains from 0.1 to 20 parts per million by weight of ammonia.

6. The process of any of claims 1 to 5 characterized in that said first feedstream is thoroughly mixed with said aqueous sulfuric acid solution and then the aqueous sulfuric acid solution and the feedstream are separated.

7. The process of claim 6 characterized in that said first feedstream is mixed with said aqueous sulfuric acid solution in a mixing eductor, the effluent from the eduction is passed through a static in-line mixer, and the effluent from the static in-line mixer is discharged into a separation column wherein the aqueous phase condenses and collects in the bottom and the olefin-containing phase is taken off overhead.

8. The process of claim 7 characterized in that the overhead from said separation column is subjected to a water wash step prior to being contacted with said methanol or said acid ion exchange catalyst.

9. The process of claim 7 characterized in that said first feedstream is pretreated with said acid solution before said feedstream is combined with said second feedstream.

## Patentansprüche

1. Verfahren zur Herstellung von aliphatischen Äthern, wobei ein erster Speisestrom, der mindestens ein Isoolefin mit 4 bis 16 C-Atomen enthält, und ein zweiter Speisestrom, der mindestens einen Alkohol ausgewählt aus primären und sekundären aliphatischen Alkoholen mit 1 bis 12 C-Atomen enthält, mit einem sauren Ionenaustauscherkatalysator in Berührung gebracht werden, dadurch gekennzeichnet, daß man den ersten Speisestrom mit verdünnter wässriger Schwefelsäure vom pH größer als 4 und kleiner als 7 bei einer Temperatur von 21 bis 49°C vermischt bevor der zweite Speisestrom in Kontakt mit dem sauren Ionenaustauscherkatalysator in Berührung gebracht wird.

2. Verfahren nach Anspruch 1, dadurch gekennzeichnet, daß der erste Speisestrom im wesentlichen aus der $C_4$-Olefinfraktion eines Kohlenwasserstoff-Crackprozesses besteht.

3. Verfahren nach Anspruch 2, dadurch gekennzeichnet, daß das Isoolefin des ersten Speisestroms im wesentlichen aus Isobutylen und der Alkohol des zweiten Speisestroms im wesentlichen aus Methanol bestehen.

4. Verfahren nach einem der Ansprüche 1 bis 3, dadurch gekennzeichnet, daß der saure Ionenaustauscherkatalysator ein saures Ionenaustauscherharz ist.

5. Verfahren nach einem der Ansprüche 1 bis 4, dadurch gekennzeichnet, daß der erste Speisestrom 0,1 bis 20 ppm Ammoniak, bezogen auf das Gewicht, enthält.

6. Verfahren nach einem der Ansprüche 1 bis 5, dadurch gekennzeichnet, daß der erste Speisestrom innig mit der wässrigen Schwefelsäure vermischt wird, und dann die wässrige Schwefelsäure und der Speisestrom getrennt werden.

7. Verfahren nach Anspruch 6, dadurch gekennzeichnet, daß man den ersten Speisestrom mit der wässrigen Schwefelsäure in einem Ejektordüsen-Strahlmischer vermischt, den Austrag aus dem Strahlmischer durch einen statischen In-Line-Mischer hindurchführt, und den Austrag aus dem statischen In-Line-Mischer in eine Trennsäule einspeist, in der die wässrige Phase kondensiert und sich am Boden ansammelt, während die olefinhaltige Phase über Kopf abgezogen wird.

8. Verfahren nach Anspruch 7, dadurch gekennzeichnet, daß der Überkopfstrom aus de Trennsäule einer Wasserwäsche unterworfen wird, bevor er mit dem Methanol oder dem sauren Ionenaustauscherkatalysator in Berührung gebracht wird.

9. Verfahren nach Anspruch 7, dadurch gekennzeichnet, daß man den ersten Speisestrom mit der wässrigen Säure vorbehandelt, bevor dieser Speisestrom mit dem zweiten Speisestrom vereinigt wird.

## Revendications

1. Procédé pour la production d'éthers aliphatiques où un premier courant d'alimentation comprenant au moins une isooléfine ayant 4 à 16 atomes de carbone et un second courant d'alimentation comprenant au moins un alcool choisi parmi des alcools aliphatiques primaires et secondaires contenant 1 à 12 atomes de carbone sont mis en contact avec un catalyseur acide d'échange d'ions, caractérisé en ce qu'on mélange le premier courant d'alimentation avec une solution aqueuse diluée d'acide sulfurique ayant un pH des plus de 4 et de moins de 7 à une température de 21—49°C avant que le premier courant d'alimentation ne soit mis en contact avec le catalyseur acide d'échange d'ions.

2. Procédé selon la revendication 1, caractérisé en ce que le premier courant d'alimentation se compose essentiellement de la fraction d'oléfines en $C_4$ d'un procédé de craquage d'hydrocarbures.

3. Procédé selon la revendication 2, caractérisé en ce que l'isooléfine dans le premier courant d'alimentation se compose essentiellement d'isobutylène et l'alcool dans le second courant d'alimentation se compose essentiellement de méthanol.

4. Procédé selon l'une quelconque des revendications 1 à 3, caractérisé en ce que le catalyseur acide d'échange d'ions est une résine acide échangeuse d'ions.

5. Procédé selon l'une quelconque des revendications 1 à 4, caractérisé en ce que le premier courant d'alimentation contient 0,1 à 20 parties par million en poids d'ammoniac.

6. Procédé selon l'une quelconque des revendications 1 à 5, caractérisé en ce que l'on melange complètement le premier courant d'alimentation avec la solution aqueuse d'acide sulfurique et l'on sépare ensuite la solution aqueuse d'acide sulfurique et le courant d'alimentation.

7. Procédé selon la revendication 6, caractérisé en ce que l'on mélange le premier courant d'alimentation avec la solution aqueuse d'acide sulfurique dans un dispositif d'extraction à melange, l'on fait passer l'effluent provenant de l'opération d'extraction à mélange a travers un mélangeur statique en ligne et l'on décharge l'effluent du mélangeur statique en ligne dans une colonne de séparation où la phase aqueuse se condense et se rassemble au bas de la colonne et la phase contenant l'olefine est recueillie en tête de colonne.

8. Procédé selon la revendication 7, caractérisé en ce que l'on soumet le produit recueilli en tête de colonne à une opération de lavage a l'eau avant sa mise en contact avec le methanol ou le catalyseur acide d'échange d'ions.

9. Procédé selon la revendication 7, caractérisé en ce que l'on soumet le premier courant d'alimentation a un traitement préliminaire avec ladite solution d'acide avant de la combiner avec le second courant d'alimentation.